# EUROPEAN PATENT APPLICATION

(11) **EP 4 763 097 A1**
(43) Date of publication of application: **24.06.2026**
(21) Application number: 25223000.8
(22) Date of filing: 12.12.2025
(51) Int. Cl.: A61B 10/02, A61B 10/06

(54) **PUNCTURE DEVICE AND PUNCTURE METHOD THEREOF**

(30) Priority: 23.12.2024 CN 202411901508
(71) Applicant: Micro-Tech (Nanjing) Co., Ltd., Nanjing, Jiangsu 210032 (CN); Zhendong, Jin, Shanghai 200433 (CN); Li, Weiwei, 400012 Chongqing (CN)
(72) Inventor: JIN, Zhendong, Shanghai, 200433 (CN); LI, Weiwei, Chongqing, 400012 (CN); XIANG, Fangyuan, Nanjing, Jiangsu, 210032 (CN); JF Herth, Felix, Nanjing, Jiangsu, 210032 (CN); ZHAO, Yongxue, Nanjing, Jiangsu, 210032 (CN); ZOU, Hui, Nanjing, Jiangsu, 210032 (CN); CAO, Ya, Nanjing, Jiangsu, 210032 (CN); TAO, Yujun, Nanjing, Jiangsu, 210032 (CN); JI, Yu, Nanjing, Jiangsu, 210032 (CN)
(74) Representative: Laine IP Oy

(57) **Abstract**

The present application discloses a puncture device and a puncture method thereof, belonging to the field of medical devices. In the puncture device of the embodiment of the present application, a position limiting member is provided on the second member, and the position limiting member is used to limit the position of the first member, so that the first member can drive the second member to move toward the third member. The catheter is connected to the second member, so that the catheter can move together with the second member. The puncture needle is connected to the first member, so that the puncture needle can be driven to move when the first member moves, and the puncture needle can drive the catheter through the tissue together during puncturing. After that, the puncture needle can be withdrawn, and the catheter can be left in the tissue for subsequent surgical treatment, thereby reducing damage to the tissue during the operation.

## Description

The present application claims the priority of Chinese patent application No. 202411901508.9 filed on December 23, 2024, which is incorporated herein by reference in its entirety.

### FIELD OF THE INVENTION

The present application belongs to the technical field of medical devices, and specifically relates to a puncture device and a puncture method thereof.

### BACKGROUND OF THE INVENTION

Biopsy puncture technique is a medical diagnostic procedure used to obtain tissue or cell samples from a patient for pathological examination. This technique employs a puncture device to accurately guide the needle tip to the lesion through the penetration of tissues, after which a sample of tissue or cells is collected. Biopsy puncture technique can be applied to lesions in various locations, such as the lungs, liver, breast, and thyroid, and is widely used in clinical practice.

Conventional biopsy puncture techniques directly acquire tissues through needle puncture and suction, which allows for relatively limited amounts of tissue extraction. Other improved methods have emerged in clinical practice, such as performing biopsies with biopsy forceps under ultrasound guidance or obtaining tissue samples using cryoprobe. However, these methods often require cutting into the tissue before applying the biopsy forceps or cryoprobe, leading to significant surgical trauma. Even when directly using biopsy forceps through the needle channel of an ultrasound needle, the opening of the biopsy forceps is small due to the size limitations of the inner lumen of the ultrasound needle core, and the sampling volume is small, which has become a notable drawback.

### SUMMARY OF THE INVENTION

### OBJECTIVES OF THE INVENTION

An objective of the embodiments of the present application is to provide a puncture device, aiming to solve the technical problem of small sampling volume in the current method of obtaining tissue using biopsy forceps through the needle channel of an ultrasonic needle. Another objective of the embodiments of the present application is to provide a puncture method.

### SOLUTIONS

According to an embodiment of the present application, a puncture device includes:
a handle mechanism provided with a channel, the handle mechanism including a first member, a second member, and a third member arranged in sequence in the length direction thereof, the channel running through the first member, the second member, and the third member in the length direction; wherein the second member is provided with a position limiting member, which is configured to limit the position of the first member so that the first member is able to drive the second member to move toward the third member;
a catheter, disposed in the channel and connected to the second member, the catheter passing through the third member along the channel and extending outside the channel; and
a puncture needle, configured to penetrate the channel and the catheter and to be connected to the first member.

In some embodiments, the first member is provided with a first locking member, and the first locking member is configured to have an unlocked state and a locked state.

When the first locking member is in the unlocked state, the first member is movable relative to the second member.

When the first locking member is in the locked state, the first locking member can be locked to the second member to fix the first member to the second member.

In some embodiments, the second member is provided with a second locking member, and the second locking member is configured to be locked to the third member to fix the second member to the third member.

The second locking element is further configured to be unlocked when pressed, so that the second member is able to move relative to the third member.

In some embodiments, the catheter includes:
a main section connected to the second member; and
a tapered section connected to an end of the main section away from the second member, wherein the outer diameter of the tapered section gradually decreases in a direction away from the main section.

In some embodiments, the inner diameter of the tapered section gradually decreases in a direction away from the main section.

In some embodiments, the inner diameter D1 at the narrowest point of the tapered section and the outer diameter D2 of the needle body of the puncture needle satisfy the following relationship: 0.5≤D1/D2≤10.

In some embodiments, the puncture device further satisfies: 0.3mm≤D1≤3.6mm.

In some embodiments, the puncture device further satisfies: 0.3mm≤D1≤1.9mm.

In some embodiments, the puncture needle is configured to contact the inner wall of the tapered section when passing through the catheter.

In some embodiments, the material of the catheter is an elastic material.

In some embodiments, the puncture device further includes:
a radiopaque part provided at an end of the catheter away from the second member.

In some embodiments, the radiopaque part is disposed between an outer wall and an inner wall of the catheter.

In some embodiments, the catheter is provided with a receiving groove, and the radiopaque part is disposed in the receiving groove.

In some embodiments, the puncture needle includes:
a needle body, configured to be able to penetrate the channel and the catheter; and
a connector, which is connected to one end of the first member away from the second member and connected to the needle body.

In some embodiments, the connector is detachably connected to the first member.

In some embodiments, a connecting portion is provided at an end of the first member away from the second member, and the connector is detachably connected to the connecting portion.

In some embodiments, the second member includes a first core rod, the first member and the position limiting member are both provided on the first core rod, and the position limiting member is configured to have an open state and a closed state.

When the position limiting member is in the open state, the position limiting member is able to move along the length direction on the first core rod.

When the position limiting member is in the closed state, the position limiting member is fixed to the first core rod to limit the position of the first member.

Accordingly, an embodiment of the present application provides a puncture method, which is applied to the puncture device described in any of the above embodiments. The puncture method includes:
limiting the position of the first member by the position limiting member, and moving the first member, so that the first member drives the second member to move toward the third member and drives the puncture needle and the catheter to pierce tissue together.

In some embodiments, the method further includes: after the puncture needle and the catheter pierce the tissue together, separating the puncture needle from the first member to withdraw the puncture needle from the catheter and the channel, so that the catheter remains in the tissue.

In some embodiments, the method further includes: moving the first member to drive the puncture needle to retract into the catheter, and passing a guide wire through the puncture needle.

In some embodiments, the method further includes: switching the first locking member on the first member to the locked state, moving the first member to drive the second member to move toward the third member, and drive the puncture needle and the catheter to pierce the tissue together.

In some embodiments, the method further includes: switching the first locking member on the first member to the locked state, pressing the second locking member on the second member, moving the second member to drive the first member to move toward the third member, and drive the puncture needle and the catheter to pierce the tissue together.

### BENEFICIAL EFFECT

The puncture device provided in the embodiment of the present application includes: a handle mechanism provided with a channel, the handle mechanism including a first member, a second member and a third member arranged in sequence in the length direction thereof, the channel running through the first member, the second member and the third member in the length direction; wherein the second member is provided with a position limiting member, which is configured to limit the position of the first member so that the first member is able to drive the second member to move toward the third member; a catheter, disposed in the channel and connected to the second member, the catheter passing through the third member along the channel and extending outside the channel; and a puncture needle, configured to penetrate the channel and the catheter and to be connected to the first member. In the puncture device of the embodiment of the present application, the position limiting member is provided on the second member and the position limiting member is used to limit the position of the first member, so that the first member is able to drive the second member to move toward the third member. The catheter is connected to the second member so that the catheter can move together with the second member. The puncture needle can be connected to the first member so that the puncture needle can be driven to move when the first member moves, so that the puncture needle can pierce through the tissue together with the catheter during puncture, and then the puncture needle can be withdrawn and the catheter can be left in the tissue for subsequent surgical treatment. In this way, the biopsy forceps can be advanced with the catheter, the sampling volume can be increased, and damage to the tissue is reduced.

The embodiment of the present application provides a puncture method, which is applied to the aforementioned puncture device, and the puncture method includes: limiting the position of the first member by the position limiting member, and moving the first member so that the first member moves the second member toward the third member, and drives the puncture needle and the catheter to pierce the tissue together. The catheter can be left in the tissue for subsequent surgical treatment, and the biopsy forceps can be advanced with the catheter, and therefore the sample volume can be increased, and damage to the tissue can be reduced.

### BRIEF DESCRIPTION OF THE DRAWINGS

In order to more clearly illustrate the solutions in the embodiments of the present application, the following briefly introduces the drawings required for the description of the embodiments. Obviously, the drawings described below are only some embodiments of the present application. For those skilled in the art, other drawings can be obtained based on these drawings without creative work.
FIG 1 is a perspective structural diagram of the puncture device provided in some embodiments of the present application.
FIG 2 is a structural diagram of the puncture device provided in some embodiments of the present application.
FIG 3 is a structural diagram of the puncture device provided in other embodiments of the present application.
FIG 4 is a partially enlarged structural diagram of area A in FIG 2.
FIG 5 is a perspective diagram of partially enlarged structure of area A in FIG 2.
FIG 6 is a cross-sectional view of a partial structure of the needle body and the catheter according to some embodiments of the present application.
FIG 7 is a cross-sectional view of a partial structure of the needle body and the catheter according to other embodiments of the present application.
FIG 8 is a cross-sectional view of a partial structure of the needle body and the catheter according to other embodiments of the present application.
FIG 9 is a flow chart of the puncture method provided in some embodiments of the present application.
FIG 10 is a flow chart of passing the guide wire with the puncture device provided in some embodiments of the present application.
FIG 11 is a cross-sectional view of the puncture device provided by some embodiments of the present application when passing the guide wire in use.
FIG 12 is a flow chart of the puncture method provided in some embodiments of the present application.

List of the reference numerals in the drawings:
100 - Handle Mechanism
101 - Channel
110 - First Member
111 - First Handle
112 - Connecting Portion
113 - First Locking Member
120 - Second Member
121 - Second Handle
122 - First Core Rod
123 - Second Locking Member
130 - Third Member
131 - Connecting Member
132 - Second Core Rod
140 - Position Limiting Member
150 - Supporting Sleeve
200 - Catheter
210 - Main Section
220 - Tapered Section
230 - Outer Wall of Catheter
240 - Inner Wall of Catheter
250 - Receiving Groove
300 - Puncture Needle
310 - Needle Body
320 - Connector
400 - Probe Assembly
410 - Probe
420 - Needle Cap
500 - Radiopaque part
600 - Guide Wire
X - Length Direction

### DETAILED DESCRIPTION OF THE EMBODIMENTS

The solutions of the embodiments of the present application will be described clearly and completely in combination with the drawings in the embodiments of the present application. Obviously, the embodiments described are only part of the embodiments of the present application, not all of the embodiments. Based on the embodiments in the present application, all other embodiments obtained by those skilled in the art without making creative efforts are within the scope of the present application.

In the description of this application, it should be understood that the terms "proximal end" and "distal end" are used with reference to the surgical operator (doctor). The "proximal end" refers to the end of a surgical tool closer to the operator, while the "distal end" refers to the end away from the operator, i.e., closer to the patient, relative to the "proximal end". Terms such as "upper," "lower," "inner," and "outer" indicate positions or locations based on those shown in the accompanying drawings and are intended solely for ease of description and simplification. They are not intended to indicate or imply that the device or apparatus referred to must have a specific orientation, be constructed, or operate in a specific orientation, and are therefore not to be construed as limitations on this application. "Multiple" means two or more, and "at least one" means one, two, or more, unless otherwise specifically defined. The terms "mounted," "connected to," and "connected with" should be broadly construed. For example, they can refer to fixed connection, detachable connection, or integral connection, they can indicate direct connection or indirect connection through intermediate media, and they can refer to internal communication between two components or interaction between two components, unless otherwise specifically defined. The terms "first" and "second" are used for descriptive purposes only and should not be understood to indicate or imply relative importance or implicitly specify the quantity of the technical features indicated. Therefore, a feature specified as "first" or "second" may explicitly or implicitly include one or more of the features.

In the description of this application, the length direction is introduced to facilitate the description of the relative positional relationships between the various components of the puncture device. The length direction is the direction along which the channel in the handle mechanism of the puncture device extends, and is also the direction of relative movement between the first, second, and third members of the handle mechanism. In the drawings of this application, an arrow marked with an X indicates the length direction X, which can be the direction from the proximal end of the handle mechanism to the distal end of the handle mechanism.

Biopsy puncture technology can be applied to lesions in different parts of the body, such as the lungs, liver, breast, thyroid, etc., and is widely used in clinical practice. For example, EBUS-TBNA (Endobronchial Ultrasound-Guided Transbronchial Needle Aspiration), which combines endoscopy and ultrasound technology, allows for direct observation and guidance of the needle to lung lesions via an ultrasound probe guided through a bronchoscope, thereby obtaining tissue samples for cytological or histological examination. Another example is digestive ultrasound endoscopic puncture and ultrasound-guided biliary selective puncture. In related technologies, the puncture device may cause damage to tissue or other instruments during actual use. For example, when passing a guide wire in ultrasound-guided biliary selective puncture, the guide wire will contact the distal blade of the needle during movement, causing the guide wire skin to be scraped. In addition, if a cryoprobe or biopsy forceps is needed for extratracheal sampling or treatment in clinical practice, an electric knife is required to cut the trachea, and then the cryoprobe or biopsy forceps is inserted through the incision, which will cause significant damage to the tracheal tissue. Even if the biopsy forceps is inserted directly through the inner needle channel of the ultrasonic needle, the opening of the biopsy forceps is small due to the size limitation of the inner lumen of the ultrasonic needle core, and the sampling volume is small.

In view of this, an embodiment of the present application provides a puncture device that can be used for puncture sampling during ultrasound endoscopy and ultrasound bronchoscopy to obtain tissue samples from the target site. It is understandable that the puncture device can also be used in other minimally invasive surgeries and is not limited to ultrasound endoscopy and ultrasound bronchoscopy. When it is applied to ultrasound-guided biliary selective puncture, it can reduce damage to the guide wire skin. When it is applied to extratracheal sampling or treatment, a catheter can be placed, and a cryoprobe or biopsy forceps can be advanced through the catheter to reduce damage to the tissue.

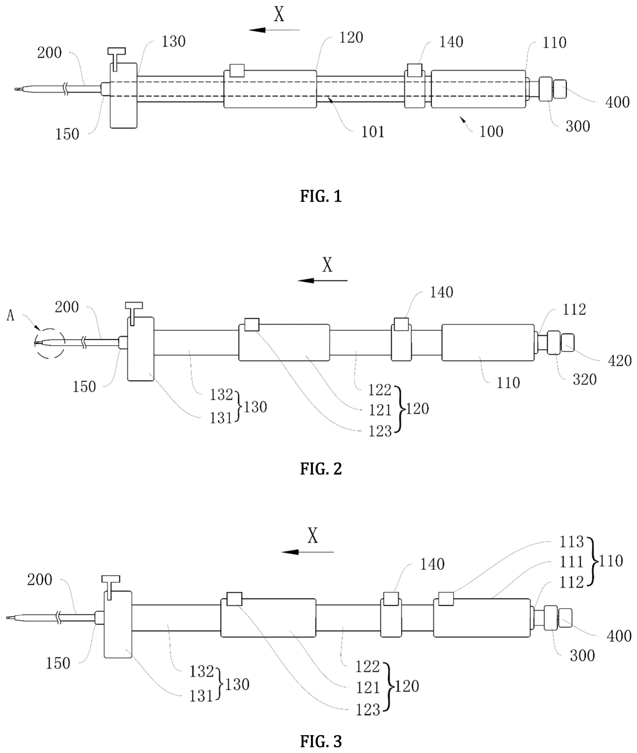

Referring to FIG 1 and FIG 2, the puncture device provided in the embodiment of the present application includes: a handle mechanism 100, a catheter 200 and a puncture needle 300.

The handle mechanism 100 is an operation and control structure of the puncture device and is usually held by a doctor or operator. The handle mechanism 100 is used to operate and control the catheter 200 and the puncture needle 300 to complete actions such as advancement, retraction, rotation and collection of tissue samples.

The handle mechanism 100 includes a first member 110, a second member 120, and a third member 130 arranged sequentially along its longitudinal direction X. The first member 110, the second member 120, and the third member 130 are arranged sequentially from the proximal end to the distal end of the handle mechanism 100 and are movably connected to each other. Through operation, the first member 110 can be moved relative to the second member 120, and the second member 120 can also be moved relative to the third member 130.

The handle mechanism 100 is provided with a channel 101 that runs through the first member 110, the second member 120, and the third member 130 in the longitudinal direction X. This channel 101 is used to route the catheter 200 and pass the puncture needle 300. By holding the handle mechanism 100, the catheter 200 and puncture needle 300 are delivered into the patient's body through the endoscope's forceps channel. The connecting member 131 of the third member 130 can be connected to the endoscope's forceps channel boss to secure it to the endoscope. The support sleeve 150 provided at the distal end of the connecting member 131 is used to pass the catheter 200.

The second member 120 is provided with a position limiting member 140, and the position limiting member 140 is configured to limit the position of the first member 110 so that the first member 110 can drive the second member 120 to move toward the third member 130.

As shown in FIG 1, in some embodiments, a position limiting member 140 can be fixed to the second member 120, so that its position on the second member 120 is fixed. When the first member 110 is controlled to move toward the second member 120 along the length direction X until it contacts the position limiting member 140, the first member 110 can apply a thrust to the position limiting member 140 toward the third member 130 along the length direction X. When the second member 120 and the third member 130 are in a relatively movable state, the position limiting member 140, under the thrust of the first member 110, can drive the second member 120 toward the third member 130. When the second member 120 and the third member 130 are in a relatively fixed state, the position limiting member 140 reacts to the first member 110 to limit position of the first member 110, causing the first member 110 to stop moving toward the third member 130 when it reaches the position limiting member 140. That is to say, the position limiting member 140 can limit the relative position of the first member 110 and the second member 120.

In some embodiments, the second member 120 includes a second handle 121 and a first core rod 122. The first core rod 122 is connected to the end of the second handle 121 near the first handle 111. The first member 110 and the position limiting member 140 are both disposed on the first core rod 122 of the second member 120. The position limiting member 140 is configured to have an open state and a closed state. When in the open state, the position limiting member 140 can move on the first core rod 122 along the longitudinal direction X. When in the closed state, the position limiting member 140 is fixed to the first core rod 122 to limit the position of the first member 110. Thus, by adjusting the position of the position limiting member 140 on the first core rod 122, the puncture distance of the puncture needle 300 can be adjusted. The position limiting member 140 can be configured as a clamp, a clip, a locking screw, or the like.

In some embodiments, the relative movable state and relative fixed state between the second member 120 and the third member 130 can be achieved by the second locking member 123 of the second member 120. When the second locking member 123 is locked, the second member 120 is fixed to the third member 130, achieving a relative fixed state between the second member 120 and the third member 130. When the second locking member 123 is unlocked, the second member 120 can move relative to the third member 130, achieving a relative movable state between the second member 120 and the third member 130. Optionally, the second locking member 123 can be a locking nut that is sleeved on the distal end of the second handle 121 of the second member 120. When the second locking member 123 is tightened, it presses the distal end of the second handle 121, so that the second locking member 123 clamps the second core rod 132 of the third member 130, and locking is achieved. When the second locking member 123 is loosened, the distal end of the second handle 121 is released, and unlocking is achieved. Optionally, the second locking member 123 can be a locking screw, which is mounted in a mounting hole of the side wall of the second handle 121. When the second locking member 123 is screwed in, its end can abut against the second core rod 132 of the third member 130 to achieve locking. When the second locking member 123 is screwed out, its end can be separated from the second core rod 132 of the third member 130 to achieve unlocking.

The second locking member 123 may also be implemented using other locking structures. In some embodiments, the second locking member 123 is configured to lock onto the third member 130 to secure the second member 120 thereto. The second locking member 123 is also configured to unlock when pressed, allowing the second member 120 to move relative to the third member 130. For example, although not illustrated, it is understood that a tooth-groove structure may be provided on the second core rod 132 of the third member 130. Specifically, the outer circumference of the second core rod 132 is provided with a plurality of grooves spaced apart along the longitudinal direction X , the grooves extending in a direction perpendicular to the longitudinal direction X. The second locking member 123 includes a base, a pressing member, and an elastic member. The base is assembled around the second core rod 132 and is fixedly connected to the second handle 121 of the second member 120. The base and the second handle 121 are configured to move along the second core rod 132 (i.e., moving relative to the second core rod 132 in the longitudinal direction X). The base is provided with a mounting hole. The pressing member is disposed within the mounting hole and is connected to the base via the elastic member. When pressed, the pressing member can move in a direction perpendicular to the longitudinal direction X, i.e., along the extension direction of the groove. The pressing member is provided with a protrusion that can be engaged into the groove of the tooth-groove structure, allowing it to lock in place by interacting with the groove. Thus, when the pressing member is not pressed, the elastic member holds the pressing member in a locked position, such that the protrusion of the pressing member is engaged into the groove of the tooth-groove structure, and the second locking member 123 cannot move relative to the third member 130 in the longitudinal direction X, thereby fixing the second member 120 to the third member 130. When a doctor or other operator applies pressure to the pressing member, the force is transmitted to the elastic member, causing it to elastically deform, and then the pressing member moves in the extension direction of the groove under pressure, driving its protrusion to move out of the groove, and unlocking is achieved and the second member 120 is able to move relative to the third member 130 in the longitudinal direction X. For another example, the second locking member 123 may include an elastic member and a latch member, wherein the middle portion of the latch member is hinged to the second handle 121, and the elastic member is fixedly connected to the second handle 121 and presses the latch member into the locked position, so that one end of the latch member can be engaged in the groove of the aforementioned tooth-groove structure. The other end of the latch member is provided with a pressing structure. When the doctor presses the pressing structure, the other end of the latch member is pressed, generating a seesaw effect, which allows it to rotate around the hinge to resist the elastic force of the elastic member, causing the end that is engaged in the groove to be disengaged from the groove, thereby achieving unlocking.

The catheter 200 is disposed within the channel 101 and connected to the second member 120. The catheter 200 extends along the channel 101, through the third member 130, and out of the channel 101. Specifically, the catheter 200 is connected to the second member 120 at one end, while the other end extends along the channel 101, through the third member 130, and out of the channel 101. Movement of the second member 120 can drive the catheter 200 to move. After the puncture device is installed in the endoscope, when the second and third members 120 and 130 are in a relatively movable state, the length of the catheter 200 extending from the endoscope can be controlled by controlling the movement of the second member 120 relative to the third member 130.

The puncture needle 300 is configured to penetrate the channel 101 and the catheter 200 and to be connected to the first member 110. In other words, the puncture needle 300 can be connected to and removed from the first member 110, i.e., they are connected in a detachable manner. By inserting the puncture needle 300 into the channel 101 and the catheter 200 and connecting it to the first member 110, movement of the first member 110 can drive the puncture needle 300 to move. The limiting action of the position limiting member 140 limits the relative position of the first and second members 110, thereby controlling the length of the puncture needle 300 extending from the catheter 200.

In the puncture device of the present embodiment, a position limiting member 140 is provided on the second member 120, which limits the position of the first member 110, allowing the first member 110 to drive the second member 120 to move toward the third member 130. By connecting the catheter 200 to the second member 120, the catheter 200 can move along with the second member 120. Therefore, after the puncture device is installed in an endoscope, the position of the second member 120 can be adjusted to control the length of the catheter 200 extending from the endoscope. By connecting the puncture needle 300 to the first member 110, movement of the first member 110 drives the puncture needle 300 to move, thereby performing puncture. Thus, the puncture needle 300 can penetrate the tissue together with the catheter 200 during puncture. When it is observed that the catheter 200 reaches the outside of the tissue under ultrasound or other conditions, the puncture needle 300 can be removed from the first member 110 and then withdrawn, leaving the catheter 200 in the tissue. The catheter 200 can be used as a pathway for subsequent treatment and surgical treatment, such as inserting a cryoprobe or biopsy forceps through the catheter 200 for sampling or other treatment, thereby increasing the amount of tissue extracted and reducing damage to the tissue.

Referring to FIG 3, in some embodiments, the first member 110 is provided with a first locking member 113. The first locking member 113 is configured to have an unlocked state and a locked state. In the unlocked state, the first member 110 is movable relative to the second member 120. In the locked state, the first member 110 is fixed to the second member 120 by the first locking member 113. In this embodiment, the locked state of the first locking member 113 can achieve fixed connection between the first member 110 and the second member 120, enabling them to operate synchronously. When the second member 120 and the third member 130 are in relative motion state, operating the first member 110 allows the puncture needle 300 and catheter 200 to pierce tissue together. By providing the first locking member 113 on the first member 110, the connection strength between the first member 110 and the second member 120 is enhanced, preventing the puncture needle 300 from retracting when subjected to force. When the first locking member 113 is set to the unlocked state, the position limiting member 140 can be used to limit the position of the first member 110 and determine the puncture distance of the puncture needle 300. The puncture and sampling functions of the puncture needle 300 itself are utilized to control the puncture needle 300 to independently penetrate the tissue via the first member 110 to achieve rapid puncture.

In the embodiment where the second locking member 123 of the second member 120 is unlocked by pressing, by providing the aforementioned first locking member 113 on the first member 110, the doctor's operation is facilitated, and simultaneous puncture of the puncture needle 300 and the catheter 200 is achieved more easily. Specifically, after the first locking member 113 is set to the locked state, the first member 110 and the second member 120 are fixedly connected and can move synchronously in the longitudinal direction X. The doctor simply grasps the second member 120 and presses the second locking member 123, and moving the second member 120 will drive the first member 110 to move with it, thereby achieving simultaneous puncture of the puncture needle 300 and the catheter 200. In other words, the doctor can perform a simultaneous puncture operation by simply grasping the second member 120 with one hand. Conversely, in the embodiment where the second locking member 123 is unlocked by pressing, if the first locking member 113 is not provided on the first member 110, the first member 110 is not locked and fixed by the first locking member 113 and is therefore movably provided on the second member 120. When the doctor grasps the second member 120 and presses the second locking member 123, moving the second member 120 toward the third member 130, the first member 110 may follow the second member 120. However, when the puncture needle 300 encounters resistance from tissue, the puncture needle 300 and the first member 110 will retract due to the resistance. Thus, to achieve simultaneous puncturing by the puncture needle 300 and the catheter 200, the doctor must use two hands to operate, with one hand holding the second member 120 and pressing the second locking member 123 to unlock, and the other hand holding the first member 110 to counteract the resistance encountered by the puncture needle 300, which increases the difficulty of operation.

Optionally, the first locking member 113 can be a locking nut that is sleeved on the distal end of the first handle 111 of the first member 110. When the first locking member 113 is tightened, it presses the distal end of the first handle 111, so that the first locking member 113 clamps the first core rod 122 of the second member 120, and locking is achieved. When the first locking member 113 is loosened, the distal end of the first handle 111 is loosened, and unlocking is achieved. Optionally, the first locking member 113 can be a locking screw, which is mounted in a mounting hole of the side wall of the first handle 111. When the first locking member 113 is screwed in, its end can abut against the first core rod 122 of the second member 120 to achieve locking. When the first locking member 113 is screwed out, its end can be separated from the first core rod 122 of the second member 120 to achieve unlocking. It is understood that the first locking member 113 can also be implemented using other locking structures, which are not limited here.

Referring to FIG 1 to FIG 4, in some embodiments, the puncture needle 300 includes a needle body 310 and a connector 320. The needle body 310 is configured to be inserted through the channel 101 and the catheter 200. The connector 320 is connected to the end of the first member 110 away from the second member 120 and is connected to the needle body 310. By inserting the needle body 310 through the channel 101 and the catheter 200 and connecting the connector 320 to the first member 110, the connection and assembly of the puncture needle 300 and the handle mechanism 100 can be completed.

In some embodiments, the connector 320 is detachably connected to the first member 110. By detaching the connector 320 from the first member 110, the needle 310 can be withdrawn from the channel 101 and the catheter 200, and the puncture needle 300 can be removed.

In some embodiments, a connection portion 112 is provided at one end of the first member 110 away from the second member 120, and the connector 320 is detachably connected to the connection portion 112. Optionally, the connection portion 112 may be a Luer connector, or may be other connection structures such as a threads or snap-fit mechanisms.

In some embodiments, the puncture device further includes a probe assembly 400. The probe 410 of the probe assembly 400 is inserted into the channel of the puncture needle 300, and the needle cap 420 of the probe assembly 400 is disposed at the proximal end of the puncture needle 300. The distal end of the probe assembly 400 is rounded. When the probe assembly 400 and the puncture needle 300 are connected, the distal end of the probe assembly 400 may extend beyond the needle tip. Providing the probe assembly 400 can reduce the impact on the needle tip of the puncture needle 300 when the puncture device is delivered into the patient's body via endoscope.

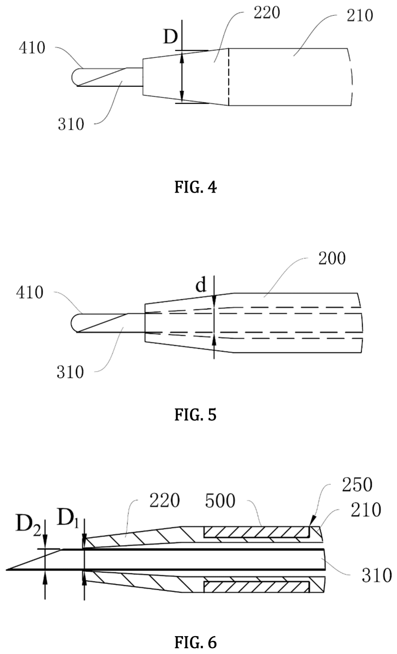

Referring to FIG 4, in some embodiments, the catheter 200 includes a main section 210 and a tapered section 220. The main section 210 is the main body of the catheter 200. The main section 210 is connected to the second member 120, and extends along the channel 101 toward the distal end of the puncture device. The tapered section 220 is connected to the end of the main section 210 away from the second member 120. The outer diameter D of the tapered section 220 gradually decreases in a direction away from the main section 210. The direction away from the main section 210 refers to the direction along the axis of the catheter 200 that is gradually away from the main section 210. In this embodiment, the outer diameter of the tapered section 220 gradually decreases as it is away from the main section 210. In other words, the tapered section 220 is thinner at the distal end and thicker toward the proximal end, giving it a generally conical shape. This configuration reduces the resistance encountered when the catheter 200 penetrates tissue, facilitating simultaneous penetration of the catheter 200 and the puncture needle 300 and minimizing tissue damage.

Referring to FIG 4 and FIG 5, in some embodiments, the inner diameter d of the tapered section 220 gradually decreases in the direction away from the main section 210. This results in a smaller distal opening of the tapered section 220, which in turn reduces the gap between the catheter 200 and the puncture needle 300 at the distal opening. This can further reduce the resistance during tissue penetration and minimize tissue damage. On the other hand, the smaller opening at the distal end of the tapered section 220 ensures a tighter fit between the puncture needle 300 and the catheter 200, facilitating the generation of static friction between them, making it easier for the puncture needle 300 to pull the catheter 200 into tissue when puncturing the tissue. Furthermore, the inner diameter d of the tapered section 220 gradually decreases, forming a generally conical guiding surface on the inner wall of the tapered section 220, which can guide the puncture needle 300 and help improve the stability and accuracy of the puncture.

Referring to FIF 6, the inner diameter of the tapered section 220 at its narrowest point is denoted as D1, i.e., the diameter of the distal opening of the catheter 200 is D1. This dimension can be measured using a measuring tool such as a vernier caliper or micrometer. The outer diameter of the needle body 310 of the puncture needle 300 is denoted as D2, i.e., the diameter of the needle body 310 of the puncture needle 300 is D2. This dimension can be measured using a measuring tool such as a vernier caliper or micrometer, or determined based on the needle gauge. For example, for a 19G needle body 310, D2 is 1.1 mm. Optionally, the following relationship is satisfied: 0.5≤D1/D2≤10. Keeping D1/D2 within this range ensures a sufficiently small gap between the puncture needle 300 and the catheter 200 at its distal opening, ensuring a close fit between them, which further reduces tissue damage, and further improves puncture stability and accuracy. Optionally, D1/D2 can be any value among 0.5, 1, 1.5, 2, 2.5, 3, 3.5, 4, 4.5, 5, 5.5, 6, 6.5, 7, 7.5, 8, 8.5, 9, 9.5, 10, or a range between any two values.

Optionally, the inner diameter D1 at the minimum inner diameter of the tapered section 220 satisfies the following relationship: 0.3 mm≤D1≤ 3.6 mm. For example, the value of D1 can be any value among 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, or a value in the range between any two values. Setting D1 within this range enables the catheter 200 to meet the requirements for EUS (Endoscopic Ultrasound) applications.

Optionally, the inner diameter D1 at the minimum inner diameter of the tapered section 220 satisfies the following relationship: 0.3 mm ≤D1≤ 1.9 mm. For example, D1 can be any value among 0.3, 0.4, 0.5, 0.6, 0.7, 0.8, 0.9, 1.0, 1.1, 1.2, 1.3, 1.4, 1.5, 1.6, 1.7, 1.8, and 1.9, or a value in the range between any two values. Setting D1 within this range enables the catheter 200 to meet the requirements of EBUS (Endobronchial Ultrasound) applications.

In some embodiments, the puncture needle 300 is configured to contact the inner wall of the tapered section 220 when it is inserted in the catheter 200. This can improve the fit between the puncture needle 300 and the catheter 200 at the opening of the catheter 200, further reducing tissue damage when they pierce the tissue together.

Optionally, the material of the catheter 200 can be an elastic material, such as medical-grade rubber, silicone, etc., so that the minimum inner diameter of the catheter 200 at the tapered section 220 can be smaller than the outer diameter of the puncture needle 300, and the puncture needle 300 can expand the catheter 200, making a tighter fit between the two components. This improves the effectiveness of simultaneous puncturing, and further reduces damage to the tissue.

Referring to FIG 6, FIG 7, and FIG 8, in some embodiments, the puncture device further includes a radiopaque part 500. The radiopaque part 500 can be configured to display images under ultrasound and/or X-ray conditions and can be made of metal or other radiopaque materials. The radiopaque part 500 is disposed at the end of the catheter 200 distal from the second member 120, i.e., at the distal end of the catheter 200. This allows accurate confirmation of the position of the catheter 200 through ultrasound or other means even after the puncture needle 300 is withdrawn.

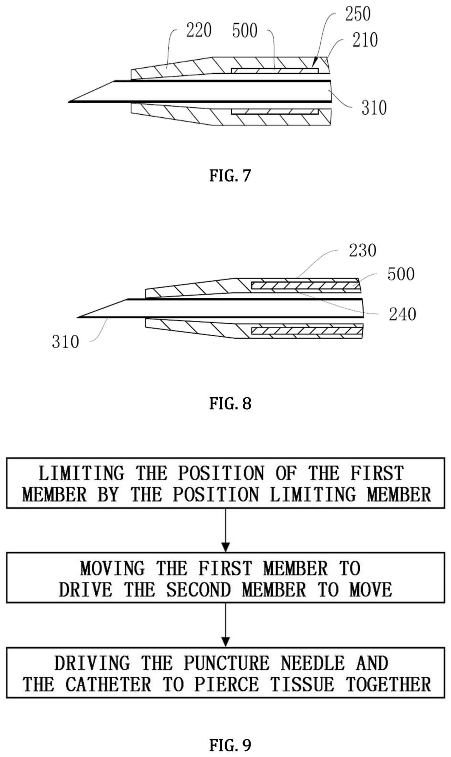

Referring again to FIG 8, in some embodiments, the radiopaque part 500 is disposed between the outer wall 230 and the inner wall 240 of the catheter 200. That is, the radiopaque part 500 is disposed within the material of the catheter 200. This does not affect the smoothness of the outer wall 230 and the inner wall 240 of the catheter 200, and can avoid contact with the puncture needle 300, the channel of endoscope, and body tissues, thereby reducing the possibility of damage to other components or tissues and improving surgical safety.

Referring to FIG 6 and FIG 7, in some embodiments, the catheter 200 is provided with a receiving groove 250, within which the radiopaque part 500 is disposed. This prevents the radiopaque part 500 from protruding, reduces the possibility of damage to other component or tissue, and enhances surgical safety. Alternatively, the receiving groove 250 may be disposed on the outer wall of the catheter 200 (as shown in FIG 6) or on the inner wall of the catheter 200 (as shown in FIG 7).

Accordingly, the embodiment of the present application further provides a puncture method applied to the puncture device of any of the above embodiments. Referring to FIG 9, the puncture method includes:
Limiting the position of the first member 110 by the position limiting member 140, and moving the first member 110, so that the first member 110 drives the second member 120 to moves toward the third member 130 and drives the puncture needle 300 and the catheter 200 to pierce the tissue together. This can reduce damage to the tissue and also allow the catheter 200 to be left in the tissue for subsequent treatment.

In some embodiments, the puncture method further includes: after the puncture needle 300 and catheter 200 pierce the tissue together, separating the puncture needle 300 from the first member 110 to withdraw the puncture needle 300 from the catheter 200 and the channel 101. This allows the catheter 200 to remain in the tissue, allowing the catheter 200 and the channel 101 to form a pathway for subsequent surgical treatments, such as inserting a cryoprobe through the catheter 200 for sampling or other treatments, or passing a biopsy forceps from the catheter 200 to acquire a tissue sample with the biopsy forceps. The larger internal channel of the catheter 200 relative to the puncture needle 300 effectively increases the sample volume that can be obtained with the biopsy forceps.

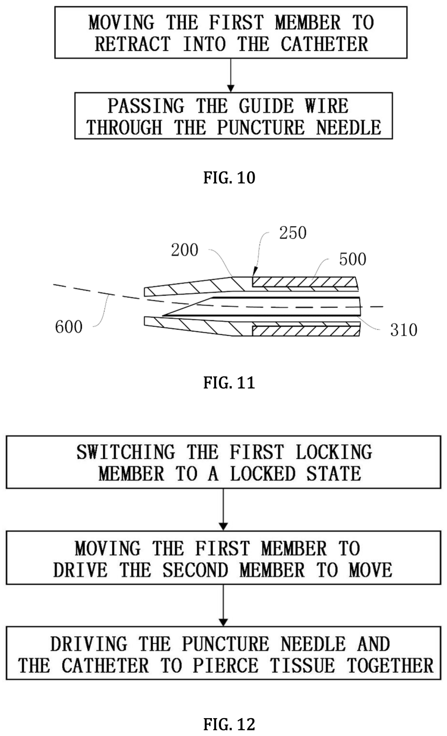

Referring to FIG 10 and FIG 11, in some embodiments, the method further includes: moving the first member 110 to drive the puncture needle 300 to retract into the catheter 200, and passing the guide wire 600 through the puncture needle 300. Specifically, when performing ultrasound biliary selective puncture, the needle tip of the puncture needle 300 is withdrawn into the catheter 200, and the guide wire 600 is passed through the puncture needle 300. Since the guide wire 600 directly contacts the catheter 200, the skin of the guide wire 600 is prevented from being cut by the needle tip of the puncture needle 300.

Referring to FIG 12, in some embodiments, when the first member 110 of the puncture device is provided with a first locking member 113, the puncture method further includes: switching the first locking member 113 on the first member 110 to a locked state, moving the first member 110 to drive the second member 120 to move toward the third member 130, and drive the puncture needle 300 and the catheter 200 to pierce the tissue together. Specifically, when the first locking member 113 is locked, the first member 110 and the second member 120 are fixedly connected, allowing the puncture needle 300 and the catheter 200 to pierce the tissue together, and the puncture needle 300 will not retract when subjected to force. When the first locking member 113 is unlocked, the position of the position limiting member 140 on the first core rod 122 can be adjusted to determine the puncture distance of the puncture needle 300, allowing for rapid puncture and retaining the puncture sampling function of the puncture needle 300. In this case, only the needle punctures, and the catheter 200 does not enter the tissue together.

In some embodiments, when the first member 110 of the puncture device is provided with a first locking member 113, and the second locking member 123 provided on the second member 120 is locked in a pressing manner, the puncture method further includes: switching the first locking member 113 on the first member 110 to a locked state, pressing the second locking member 123 on the second member 120, and moving the second member 120 to drive the first member 110 to move toward the third member 130, and drive the puncture needle 300 and the catheter 200 to pierce the tissue together. In this way, the doctor only needs to hold the second member 120 with one hand to perform the puncture operation, which is convenient for the surgical operation.

In the above embodiments, the description of each embodiment has its own focus. For parts that are not described in detail in a certain embodiment, reference can be made to the relevant descriptions of other embodiments.

Detailed description to the puncture device and puncture method provided in the embodiments of the present application has been made in the above, and specific examples are used to illustrate the principles and implementation methods of the present application. The description of the above embodiments is only used to help understand the technical solutions and core ideas of the present application. Those skilled in the art should understand that they can still modify the solutions recorded in the aforementioned embodiments, or replace some of the features therein with equivalents, and these modifications or replacements do not cause the essence of the corresponding solutions to deviate from the scope of the embodiments of the present application.

## Claims

1. A puncture device, comprising:
A handle mechanism (100) provided with a channel (101), the handle mechanism (100) comprising a first member (110), a second member (120), and a third member (130) arranged in sequence in a length direction (X) thereof, the channel (101) running through the first member (110), the second member (120), and the third member (130) in the length direction (X); wherein the second member (120) is provided with a position limiting member (140), which is configured to limit a position of the first member (110) so that the first member (110) is able to drive the second member (120) to move toward the third member (130);
a catheter (200), disposed in the channel (101) and connected to the second member (120), the catheter (200) passing through the third member (130) along the channel (101) and extending outside the channel (101); and
a puncture needle (300), configured to penetrate the channel (101) and the catheter (200) and to be connected to the first member (110).

2. The puncture device according to claim 1, wherein the first member (110) is provided with a first locking member (113), and the first locking member (113) is configured to have an unlocked state and a locked state;
when the first locking member (113) is in the unlocked state, the first member (110) is movable relative to the second member (120); and
when the first locking member (113) is in the locked state, the first locking member (113) is locked to the second member (120) to fix the first member (110) to the second member (120).

3. The puncture device according to claim 2, wherein the second member (120) is provided with a second locking member (123), and the second locking member (123) is configured to be locked to the third member (130) to fix the second member (120) to the third member (130) ; and
the second locking member (123) is also configured to be unlocked when pressed, so that the second member (120) is able to move relative to the third member (130).

4. The puncture device according to any one of claims 1 to 3, wherein the catheter (200) comprises:
a main section (210) connected to the second member (120); and
a tapered section (220) connected to an end of the main section (210) away from the second member (120), wherein the outer diameter of the tapered section (220) gradually decreases in a direction away from the main section (210).

5. The puncture device according to claim 4, wherein the inner diameter of the tapered section (220) gradually decreases in a direction away from the main section (210).

6. The puncture device according to claim 4, wherein the puncture needle (300) is configured to contact the inner wall of the tapered section (220) when passing through the catheter (200).

7. The puncture device according to any one of claims 1 to 3, wherein the puncture needle (300) comprises:
A needle body (310), configured to be able to penetrate the channel (101) and the catheter (200);
A connector (320), which is connected to an end of the first member (110) away from the second member (120) and is connected to the needle body (310).

8. The puncture device according to claim 7, wherein the connector (320) is detachably connected to the first member (110).

9. The puncture device according to claim 8, wherein a connecting portion (112) is provided at an end of the first member (110) away from the second member (120), and the connector (320) is detachably connected to the connecting portion (112).

10. The puncture device according to any one of claims 1 to 3, wherein the second member (120) includes a first core rod (122), the first member (110) and the position limiting member (140) are both provided on the first core rod (122), and the position limiting member (140) is configured to have an open state and a closed state;
when the position limiting member (140) is in the open state, the position limiting member (140) is able to move along the length direction (X) on the first core rod (122); and
when the position limiting member (140) is in the closed state, the position limiting member (140) is fixed to the first core rod (122) to limit the position of the first member (110).

11. A puncture method applied to the puncture device according to any one of claims 1 to 10, wherein the puncture method comprises:
limiting the position of the first member (110) by the position limiting member (140), and moving the first member (110), so that the first member (110) drives the second member (120) to move toward the third member (130), and drives the puncture needle (300) and the catheter (200) to pierce tissue together.

12. The puncture method according to claim 11, further comprising: after the puncture needle (300) and the catheter (200) pierce the tissue together, separating the puncture needle (300) from the first member (110) to withdraw the puncture needle (300) from the catheter (200) and the channel (101), so that the catheter (200) remains in the tissue.

13. The puncture method according to claim 11, further comprising: moving the first member (110) to drive the puncture needle (300) to retract into the catheter (200), and passing a guide wire (600) through the puncture needle (300).

14. The puncture method according to claim 11, further comprising: switching a first locking member (113) on the first member (110) to a locked state, moving the first member (110) to drive the second member (120) to move toward the third member (130), and drive the puncture needle (300) and the catheter (200) to pierce the tissue together.

15. The puncture method according to claim 11, further comprising: switching a first locking member (113) on the first member (110) to a locked state, pressing a second locking member (123) on the second member (120), and moving the second member (120) to drive the first member (110) to move toward the third member (130), and drive the puncture needle (300) and the catheter (200) to penetrate the tissue together.
